# EUROPEAN PATENT APPLICATION

(11) **EP 4 768 692 A1**
(43) Date of publication of application: **01.07.2026**
(21) Application number: 24383488.4
(22) Date of filing: 31.12.2024
(51) Int. Cl.: E21B 25/16, E21B 47/026, G01B 11/24, G01N 33/24, G06T 17/00

(54) **SYSTEM AND METHOD FOR ANALYSING AND LOGGING A CORE SAMPLE**

(71) Applicant: Stockholm Precision Tools S.L., 29196 Málaga (ES)
(72) Inventor: RAMIREZ OZUNA, Orlando Rene, Málaga (ES)
(74) Representative: Isern Patentes y Marcas S.L.

(57) **Abstract**

The present invention provides a system (1) for analysing and logging core samples (2), which include planar structures and a reference line (BOH) indicating their original rotational position. The system has a portable device (5) to capture 2D images, identify planar structures, generate fitted ellipses, calculate alpha, beta and gamma angles, and build 3D models. In addition, it can determine the dip and dip direction by projecting them on stereographic diagrams in real time. The portable device (5) allows the user to interactively adjust the generated ellipses and see results in real time. An advanced algorithm ensures accurate structure identification, even in complex samples. The generated data, which is stored in a cloud-based database, includes images, calculations and 3D models, facilitating real-time geological collaboration.

## Description

### Technical field of the invention

The invention relates to a system and method for analysing core samples taken from exploratory boreholes, wherein the system and method, based on an image of the core sample taken or captured by a portable device enabled for this purpose, allows the analysis of the discontinuities, if any, that are visible in said sample, obtaining from this analysis data on the location and angles of these discontinuities in relation to the core axis and its original position in the borehole.

### State of the art

The exploration, definition and eventual successful extraction of ore bodies, as well as the safe construction of large structures such as skyscrapers, dam walls and tunnels, depend to a large extent on a thorough understanding of the geology of the area. This understanding of the subsoil is developed through surface geological mapping combined with geophysical and geochemical studies. However, without reliable and accurate subsoil data, the predictive ability of the models used is significantly limited.

To obtain accurate subsoil data, various boring methods, such as diamond boring, are used to extract deep rock samples in the form of continuous cylinders called cores. These core samples, or cores, are analysed by a process called core logging, in which their characteristics are measured and described. The data collected is processed and analysed to refine the subsoil models and determine parameters such as the size and grade of the ore body, possible hidden deposits, blasting requirements, rock mass stability and ground behaviour under stress.

Core logging involves measuring and describing numerous characteristics, such as lithological and alteration intervals, rock densities, compressive and tensile strengths, and the shear strength of discontinuities. It also includes determining the orientation of planar structures such as layers, fractures and faults in relation to the core axis. Other tasks include identifying missing sections of core, defining lengths of intact versus fragmented rock, and assessing borehole quality.

Traditional methods of core logging are inefficient as they rely on manual data entry on standardised or customised forms. This manual process, followed by transcription into digital databases, leads to errors in editing, validation and auditing, which compromises the boring programme and the accuracy of the terrain models.

In practice, only two steps are required to prepare a core for measurements. First, the intersection of the vertical geographic plane with the core is marked as a "Bottom of Hole Line" (BOH). Second, discontinuities in the core are identified where, from these discontinuities, data can be obtained on the location and angles of the discontinuities in relation to the core axis and the original position of the core in the borehole.

Discontinuities are identified by visual inspection. Relative orientations are determined using a protractor or goniometer. For planar structures, two angles are required, and for alignments, two to three angles are measured. These angles are called Alpha (α), Beta (β) and Gamma (γ). The intersection of any planar structure with the borehole core forms an ellipse defined by its major and minor axes.

The Alpha (α) angle is defined as the acute angle between the bore axis and the major axis of the resulting ellipse. The Beta (β) angle is measured from the orientation line (BOH) to the lower apical point of the intersection ellipse, varying from 0° to 360° according to the right-hand rule. With respect to the Gamma (γ) angle, this is the angle between the lower apical point and the projection of the lineation on the associated plane (for example, fault or fold planes). It can also be defined using the intersection of the BOH line with the plane of the lineation, always following the right-hand rule. This method of obtaining angles facilitates the geometric characterisation of geological structures and alignments in bored cores.

In view of the above, the state of the art has provided solutions for obtaining the orientation angles of geological structures, which are documented in relation to the borehole and depth of the sample, as well as including qualitative characteristics. One of the methods used to measure these angles is the goniometer, which consists of a short tube of transparent material with angle lines and degree scales on its outer surface. By placing the core inside the tube and moving it along the sample, the planar structures can be measured and documented by comparing the angles with the marks on the tube.

Alternatively, European patent document EP3256851 B1 describes a portable device for recording the orientation and disposition of geological structures in borehole cores, the device comprises an orientation arrangement provided with an accelerometer, gyroscope and optionally a magnetometer, to measure angles with respect to the core axis, an alignment laser that projects visual references on the surface of the core to identify planes and faults, an optical sensor that measures displacements with high accuracy, data integration means prepared to transfer information to software for 3D models and stereographic analysis, and a user interface with buttons to record and adjust measurements in real time.

While the goniometer requires essentially manual work to carry out the measurements, which slows down and makes the logging of the cores imprecise, the device mentioned in the patent is a device especially made to carry out the loggings, it being necessary to link same to an external terminal to view the logged data, which is expensive and which, in the event of a breakdown, may not be easily replaceable, nor are the measurements scalable to other devices.

Therefore, systems and methods for core analysis and core logging are required that overcome the disadvantages mentioned above.

### Description

To overcome the drawbacks found, the present invention provides a system for analysing and logging core samples, wherein each core sample comprises planar structures and a reference line (Bottom of the Hole, BOH) which is indicative of the rotational position that the core sample had at its original location in the downhole, as described in claim 1.

Particular embodiments of the system of the invention are detailed in the claims that are dependent on claim 1.

According to a first embodiment, the system comprises a tray for positioning the core sample, a carrier intended to be coupled to the tray and positioned above the core sample, the carrier being configured for coupling a portable device intended to capture an image of the core sample to identify planar structures in the core samples, calculate key structural parameters such as alpha, beta and gamma angles, and build accurate three-dimensional models of the analysed core samples.

In this regard, said portable device comprises image capturing means configured to capture an image of the core sample and processing means configured to, from the captured image of the core sample:
- identify a planar structure to be analysed;
- generate an ellipse related to the planar structure;
- calculate the alpha, beta and gamma angles of the core sample from the generated ellipse; and
- generate a 3D model of the core sample, based on a diameter of the core sample and the generated ellipse.

In one embodiment, the portable device comprises display means configured for a user to view the captured image of the core sample, wherein the calculated alpha, beta and gamma angles are instantaneously shown by the display means and wherein the generated ellipse is compared in real time to the intersection ellipse generated by the intersection between the planar structure and the surface of the core, such that the processing means are configured to adjust or fine-tune the generated ellipse based on a possible offset from the intersection ellipse.

Alternatively, as the generated ellipse is projected or displayed by the display means, the user can compare, in real time, said generated ellipse with the ellipse seen from the planar structure itself, called, as mentioned above, the intersection ellipse, and, if necessary, interactively make adjustments, selecting specific points of the intersection ellipse such that the processing means recalculate the generated ellipse by re-fitting based on such points, refining or readjusting said generated ellipse.

In an alternative embodiment, the system is also designed to calculate the dip and dip direction of planar structures present in the core sample. These calculations, based on the alpha, beta and gamma angles generated from the fitted ellipse, are projected onto stereographic diagrams.

In an alternative embodiment, the portable device is intended to capture the image and process it in real time. This includes the generation of the ellipse related to the identified planar structure and the interactive comparison of this ellipse with the theoretical intersection ellipse between the planar structure and the surface of the core. Furthermore, "capturing an image" indicates that the portable device is configured to, advantageously, project, by means of display means, in real time the image of the core sample and project on it the ellipse and the calculated angles.

In this respect, the stereographic diagrams are projected in real time, which facilitates their interpretation and detailed geological analysis.

In other embodiments, the captured image is a 2D photograph of the core sample, and all of the above calculations, including alpha, beta, gamma angles, as well as dip and dip direction, are performed on said photograph. In addition, the 3D model of the core sample is generated from this photograph.

The advanced segmentation algorithm implemented in the portable device enables automatic identification of planar structures based on differences in colour or texture in the captured images. This algorithm, which includes a method based on RANSAC, guarantees an accurate fit of the generated ellipse, even for complex samples.

In particular, the cloud-based storage includes a structured database designed to log the captured images, calculated angles and coordinates of the analysed structures. This database is equipped with automatic backup tools to ensure data security and accessibility. It also allows integration with other geological platforms, facilitating more detailed three-dimensional models and real-time collaboration between geographically distributed teams.

In other embodiments, the system of the invention comprises additional elements that enhance its functionality, such as sliding rails to enable movement of the carrier through the tray, displacement sensors to log the position of the portable device relative to the core sample, and communication means that enable data to be transmitted to the server for storage and further processing. The data generated can therefore be synchronised and stored on a server or cloud-based platform, facilitating remote access and collaborative analysis.

Thus, the system of the invention provides a comprehensive and highly efficient tool for geological analysis, significantly improving the accuracy, speed and accessibility of the data generated. This invention is particularly useful in exploration and production applications, where data quality and reliability are critical for decision making.

### Brief description of the figures

The foregoing and other advantages and features will be better understood based on the following detailed description of several embodiments in reference to the attached drawings, which must be interpreted in an illustrative and non-limiting manner and in which:
- Figure 1 is a general view of the system showing the tray, an elongated core sample, the carrier arranged in the tray with the portable device positioned above the sample.
- Figure 2 is an alternative view of the system showing a version of the carrier with wheels that slide on rails linked to the tray.
- Figure 3 is a view of the image captured by the portable device of the core sample, showing the ellipse generated from the planar structure.

### Detailed description of an exemplary embodiment

The following detailed description provides numerous specific details as examples to provide a thorough understanding of the relevant teachings. However, it will be apparent to those skilled in the art that the present teachings can be implemented without such details.

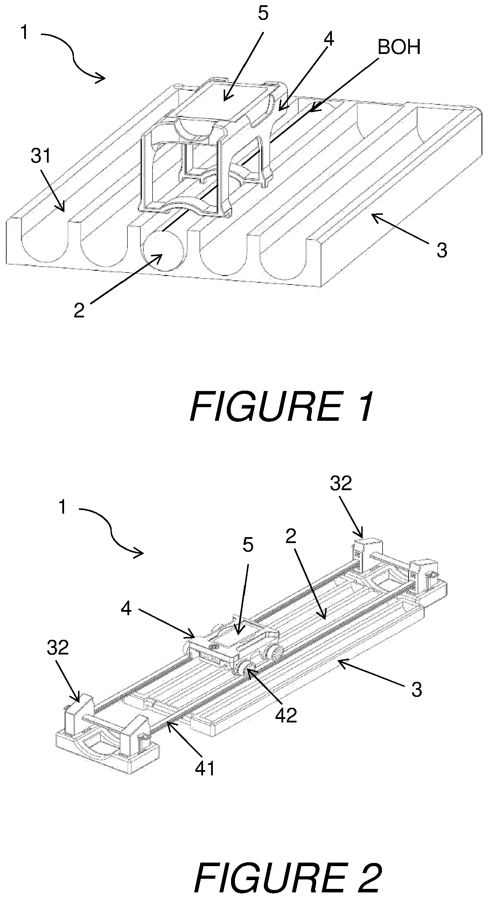

Figures 1 and 2 show a system (1) for analysing and logging core samples that is designed to address constraints in geological exploration. This system includes specific components that work together to provide accurate data and efficient automated processing.

As shown in Figure 1, the system (1) comprises a tray (3) configured as a tray in the preferred embodiment including several recesses or grooves in which multiple elongated core samples (2), hereinafter cores (2), are arranged. These grooves stabilise the cores (2) during the analysis process and ensure that the reference line (BOH) of each core is visible to ensure accurate alignment.

A carrier (4) is arranged on the tray (3) and is configured to support a portable device (5) and allows it to be moved along the tray (3) to analyse each of the elongated core samples (2). In this detailed embodiment, the portable device (5) corresponds to a smartphone (5) or the like equipped with means suitable for image capture, data processing, viewing by a user of the captured images and communication means for data transmission/reception. In particular, a smartphone-type device (5) comprises a processor, a photography camera and/or video camera, and a display or screen and antennas or similar for wireless data transmission (Wi-Fi, Bluetooth^{®}, among others).

Preferably, the system (1) comprises displacement sensing means configured to log the exact position of the carrier (4) and/or the portable device (5) relative to the core sample (2) and/or the tray (3), such that the portion of the core sample being analysed can be accurately logged. In the alternative embodiment illustrated in Figure 2, the system comprises rails (41) linked to the tray (3), in particular arranged above the cavity (31) where the sample (2) is deposited. The rails (41) are coupled to bases (32) each arranged at the ends of the cavity (31), the bases (32) in turn being movable through the tray (3), such that both the rails (41) and said bases can be located in each of the cavities (31) of the tray (3). Thus, the carrier (4) is slidably coupled to said rails (41), such that it is able to move along the same. In the illustrated embodiment, the carrier has wheels (42) that rest on and slide along the rails (41).

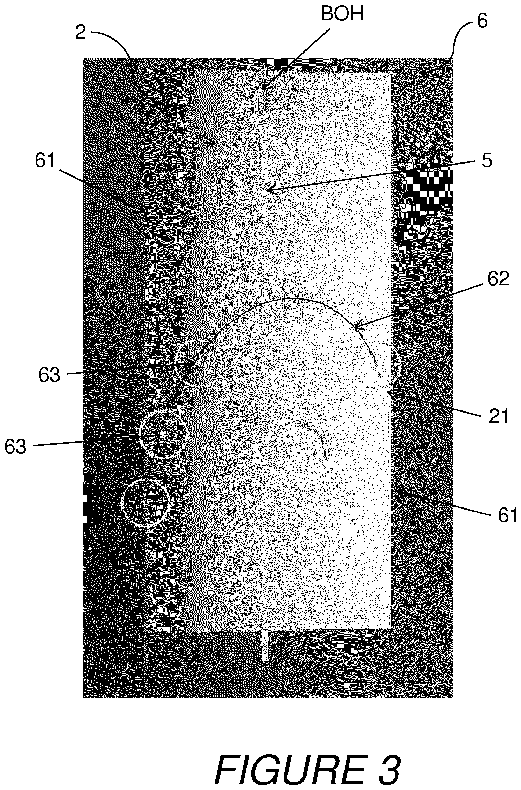

As shown in Figure 3, the smartphone (5) captures an image (6) of each core sample (2). This image (6) serves as the basis for all subsequent calculations. Once the image (6) is captured, the processing means (not shown) of the smartphone (5), hereinafter processor, is configured to identify the planar structures (21) in each sample (2) and perform calculations based on this detection.

This processing includes the generation of a fitted ellipse (62) that represents the intersection of the planar structure (21) with the surface of the core (2). From this ellipse (62), the alpha (α), beta (β) and gamma (γ) angles are calculated to determine the dip and dip direction of the planar structure.

In more detail, and as illustrated in Figure 3, from the captured image (6), the processor is configured to
- detect the edges of the sample (2), and generate a contour line (61);
- detect the reference line (BOH);
- detect the planar structure (21) to be analysed;
- generate an ellipse (62), related to the intersection of a planar structure of the sample (2); and
- from the ellipse (62), calculate the alpha (α), beta (β) and gamma (γ) angles indicative of the orientation of geological discontinuities such as fractures or veins in relation to the sample (2) and hence to the structure of the source rock.

Additionally, from the ellipse (62), the processor is configured to perform fundamental structural calculations, such as dip and dip direction of the sample (2).

In relation to the detection of the planar structure (21), the processor is configured to analyse the captured image (6) and separate different regions of interest, such as surfaces or discontinuities in the sample (2). In this regard, the processor applies image segmentation, clustering algorithms and outlier detection methods. It is noted that when the planar structure (21) (for example, intersecting ellipse) has been previously marked by the geologist, the processor applies a modified RANSAC (Random Sample Consensus) algorithm.

Alternatively, the processor is configured to apply colour segmentation independently or in combination with clustering algorithms for detection of the planar structure (21).

Additionally, in yet another alternative, in the image (6) captured and projected on the screen, the user can indicate points of the planar structure on the screen, such that the processor complements the detection of the planar structure (21) with such chosen points.

In another alternative, for the generation of the ellipse (62), the processor is configured to detect points on the planar structure (21) and generate by interpolation an ellipse that includes these detected points. In alternative embodiments, the generated ellipse (62) is displayed on the screen and the user can select specific points (63) on the planar structure (21) such that the processor resets or recalculates the ellipse (62) based on those specific points (63).

Furthermore, with regard to the calculation of the alpha (α), beta (β) and gamma (γ) angles, the processor is configured to generate and solve systems of equations by processing and transforming three-dimensional vectors, obtaining the necessary coefficients to define the geological plane in which the planar structure (21) is located. Once the coefficients of the plane have been solved, the processor calculates these angles using quick geometric formulas.

Likewise, for the dip and dip direction calculation, the processing means are configured to generate matrices that describe how the core sample (2) is oriented in space, based on the calculated Alpha (α) and Beta (β) angles and the azimuth, and the dip which are provided manually. These matrices are dynamically built using specific functions for each component of the rotation. The structural orientations are calculated by transforming the polar axis of the core sample (2) into a three-dimensional space, considering its initial alignment and applying the necessary rotations. Thus, the dip is calculated from the angle between the structural plane and a horizontal plane by calculating the maximum slope using trigonometric formulas based on the transformed vector. The dip direction is obtained by the angular orientation of the resultant vector relative to north, adjusting its position within 360°. Finally, functions such as sine, cosine and inverse tangent are applied to calculate angles in radians, which are then converted to degrees.

In yet another alternative, the processing means are implemented with an artificial intelligence trained and configured to, from the captured image, identify the planar structure, generate an ellipse, and perform the calculations of the alpha (α), beta (β) and gamma (γ) angles, as well as the dip and dip direction.

The smartphone (5) is also configured to project, in real time, the captured image together with the generated ellipse (62) and the calculated angles. Alternatively, when the photograph is captured, the smartphone (5) can superimpose the ellipse and the calculated results on the image.

In another alternative embodiment, the processing means are configured to calculate poles and planes from the data obtained in the Alpha (α), Beta (β), dip and dip direction values, then they are projected into a three-dimensional system that is translated into the two-dimensional space of a stereographic graph. In that sense, the processing means use the Schmidt equiangular projection to represent the poles and planes in the stereographic graph. The graphs include interactive tools to visualise or disable elements such as poles and planes, avoiding visual overload when handling large amounts of data. Thus, it is possible to compare the intersection between the ellipses generated by the data and the projected geological structures, providing instant visual validation.

The 3D model of the core sample (2) is generated using the information obtained from the 2D photograph, together with the diameter of the sample and ellipse data. This model enables a detailed analysis and facilitates geological interpretation.

The generated data is synchronised with a cloud-based platform (not shown), which acts as a secure and structured storage medium. This platform includes a database to log the images, the calculations made and the associated coordinates. It also allows remote analysis and collaboration between different users, ensuring data accessibility.

It is important to note that the system (1) and, in particular, the core sample (2) to be analysed is not limited by any particular diameter, such that both the system (1) and the lessons learned about it are applicable to all possible diameters of core samples (2), including the existing standards B, N, H, P.

In this exemplary embodiment the system demonstrates its ability to significantly improve the accuracy and efficiency of core sample analysis, providing advanced tools for geological exploration and production.

## Claims

1. A system (1) for analysing and logging core samples (2), wherein the core sample (2) comprises a planar structure and a reference line (BOH) indicative of the rotational position that the core had at its original location in the downhole, the system (1) comprising:
- a tray (3) configured to house the core sample (2), wherein the reference line (BOH) is visible;
- a carrier (4) arranged in the tray (3) above the core sample (2); and
- a portable device (5) coupled to the carrier (4), and comprising image capturing means configured to capture an image of the core sample and processing means configured to, from the captured image of the core sample:
- identify a planar structure to be analysed;
- generate an ellipse related to the planar structure;
- calculate the alpha, beta and gamma angles of the core sample (2) from the generated ellipse; and
- generate a 3D model of the core sample (2), also based on a diameter of the core sample (2) and the generated ellipse.

2. The system according to claim 1 wherein the portable device (5) comprises display means configured for a user to view the captured image of the core sample (2), wherein the calculated alpha, beta and gamma angles are instantaneously displayed by the display means, and wherein the generated ellipse is compared in real time to the intersection ellipse between the planar structure and the surface of the core, such that the processing means are configured to adjust the generated ellipse based on an offset from the surface of the core.

3. The system according to claim 2, wherein the generated ellipse includes interactive visual indicators presented by the display means that allow the user to verify the accuracy of the fitting prior to calculating the alpha, beta and gamma angles of the core sample.

4. The system according to any of the preceding claims, wherein the processing means of the portable device (5) are configured to apply a segmentation algorithm prepared to automatically identify the planar structure from differences in colour or texture in the captured image.

5. The system according to claim 4, wherein the segmentation algorithm includes a method based on the RANSAC algorithm prepared to fit the generated ellipse to the identified planar structure.

6. The system according to any of the claims 2 to 5, wherein the processing means are additionally configured to, from the calculation of the alpha, beta and gamma angles, calculate the dip of the sample and the dip direction of the sample, and wherein the processing means are configured to prepare interactive stereographic graphs (Stereonet) of the calculations made.

7. The system according to any of the preceding claims comprising rails (41) linked to the tray (3), wherein the carrier (4) is slidably coupled to said rails (41), such that it is able to move along the same.

8. The system according to any of the preceding claims, comprising displacement sensing means configured to log the exact position of the carrier (4) and/or the portable device (5) relative to the core sample (2) and/or the tray (3).

9. The system according to any of the preceding claims, wherein the portable device (5) comprises communication means configured to synchronise and transmit the calculations to a server and perform further analysis remotely.

10. The system according to claim 9, wherein the server comprises a structural database for logging the captured images, the calculated angles and the coordinates of the analysed core.
